(19)

Europäisches
Patentamt
European
Patent Office
Office européen
des brevets

(11)  **EP 3 051 450 A1**

(12)  **EUROPEAN PATENT APPLICATION**

(43) Date of publication:
**03.08.2016   Bulletin 2016/31**

(51) Int Cl.:
***G06F 19/22*** *(2011.01)*   ***C12Q 1/68*** *(2006.01)*

(21) Application number: **15153406.2**

(22) Date of filing: **02.02.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **Applied Maths
9830 Sint-Martens-Latem (BE)**

(72) Inventors:
• **Pouseele, Hannes
8400 Oostende (BE)**

• **Janssens, Koen
9040 Gent (BE)**

(74) Representative: **Gevers Patents
Intellectual Property House
Holidaystraat 5
1831 Diegem (BE)**

Remarks:
Claims 16-18 are deemed to be abandoned due to non-payment of the claims fees (Rule 45(3) EPC).

(54)   **Method of typing nucleic acid or amino acid sequences based on sequence analysis**

(57)   The invention relates to a method for determining the presence or absence of predefined alleles in a set of read sequences, comprising:

a) defining a k-mer, which is a nucleic acid sequence of a length "k", and a k-mer space which consists of all the permutations of nucleic acids ($4^k$) with the selected length "k";
b) for each predefined allele, determining which k-mer is present in said predefined allele, thereby obtaining an allele-associated k-mer set;
c) providing a set of read sequences;
d) for each predefined allele and allele-associated k-mer set, determining the number of occurrences of each k-mer in said set of read sequences;
e) filtering the determined number of occurrences of each k-mer; by resetting this number of occurrences of each k-mer to 0 if: i) the total number of occurrences is below a predefined threshold, ii) the total number of occurrences in the forward direction is below a predefined threshold, or iii) the total number of occurrences in the reverse direction is below a predefined threshold;
f) determining the presence or absence of each predefined allele in the read sequences based on the filtered number of occurrences of each k-mer obtained from step e).

**EP 3 051 450 A1**

**Description**

**Technical field of the invention**

The technical fields of the present invention are bioinformatics, genetics, microbiology, epidemiology, and evolutionary studies.

**Technical background of the invention**

The present invention is situated in the field of molecular bacterial typing and subtyping. The goal of any (sub)typing method is to characterize bacteria beyond the species (or subspecies) level, and to group individual isolates together in a meaningful way. Ideally, a typing method must have sufficient typeability (the ability to type isolates unambiguously), reproducibility and transportability (the ability to perform the method reproducibly in a fully compatible fashion in different laboratories at different times), must be relatively easy to perform, and have sufficient discriminatory power [1].

The ability to do this quickly and reliably is the cornerstone of laboratory-based surveillance [21]. Isolates that have indistinguishable subtypes are more likely to have originated from a common source than those with different subtypes. This concept forms the basis for applying molecular subtyping to bacterial pathogens for surveillance, outbreak detection and outbreak response. To be suitable for laboratory-based surveillance and outbreak detection, a subtyping method should be assessed against several key performance criteria [21]: typeability, reproducibility, discriminatory power and epidemiological concordance. These criteria must be assessed using an epidemiologically relevant panel of isolates from geographically as diverse a region as where the method is to be applied. Additional criteria to assess method feasibility include speed, through-put, cost, ease of use, objectivity, versatility and portability. The importance of these criteria is further emphasized for the successful application of a subtyping method to interlaboratory surveillance [22]. In particular in the novel field of subtyping based on short read sequencing data, there is a need for efficient and reliable analysis strategies that are as close and truthful to the raw data as possible.

The present invention solves at least one of the shortcomings mentioned above, or fulfills at least one of the requirements mentioned supra.

Molecular typing methods can be categorized into two groups, phenotypic and genotypic methods. In the 1980s only phenotypic techniques were available. Phenotypic assays had low reproducibility, low typeability and insufficient discriminatory power to apply to epidemiological studies. In this epidemiologic context, genotypic techniques with better typeability and discriminatory power replaced phenotypic methods during the 1990s [1]. Genotypic methods are divided into i) band-based methods, which involve analysis of DNA banding patterns by gel electrophoresis, also known as gel-based typing methods; and ii) sequence-based methods, which rely on DNA sequence analysis.

The most commonly used band-based methods were restriction endonuclease analysis (REA), pulsed-field gel electrophoresis (PFGE), capillary or conventional PCR ribotyping, MultiLocus Enzyme Electrophoresis (MLEE), and multilocus variable-number tandem repeat analysis (MLVA), whereas the most frequently used sequence-based genotyping method was multilocus sequence typing (MLST, or, as a variant, single locus sequence typing).

In the early 1990s, the only method that was suitable for studying the global or long-term spread of strains was MLEE. This method identifies variants of the gene products of 10-20 housekeeping genes (genes encoding basic metabolic functions) using electrophoresis of cell extracts on starch gels, followed by detection using specific enzyme stains. In most bacterial populations there are a number of variants of each enzyme that differ in charge, reflecting slight differences in the amino acid sequences of the proteins, and thus of their corresponding gene sequences, and these variants are assigned as different alleles. Isolates with the same alleles at each housekeeping locus are assumed to be very closely related and are assigned to the same clone (strain).

However, MLEE, like the other band-based methods, had major deficiencies, notably the great difficulty in comparing results from one laboratory with those from another. A subsequent step was to convert the method into a DNA sequence-based procedure so that the different DNA sequences at each housekeeping gene (locus) in a pathogen were assigned directly as different alleles, rather than assigning alleles indirectly using differences in the electrophoretic mobilities of their gene products on starch gels. This simple modification has huge advantages as sequence data are unambiguous and easily compared between laboratories, and, moreover, the alleles at each locus, and the allelic profiles and isolate information for each pathogen, could be stored in online databases that can be interrogated via the internet. It also required the use of fewer loci than MLEE as sequencing identifies more alleles per locus, and provides a simple nomenclature as each different allelic profile can be assigned as a different sequence type (ST), which provides a convenient strain descriptor [2].

Multi-locus sequence typing (MLST) was introduced in 1998 [23] and has proved to be a highly successful approach to molecular typing. MLST schemes for most of the major bacterial pathogens were soon developed, with seven housekeeping loci becoming established as the norm. This sequence-based typing method relies on sequencing of DNA fragments approximately ranging from 300 to 500 bp and representing seven house-keeping genes (MLST 7HG). Sequence variants for each housekeeping gene are assigned a distinct allele number and the combination of seven allele numbers (allelic profile) provides a sequence type (ST). MLST generates high-throughput sequence data that can be uploaded from laboratories worldwide to a common web database [4]. This facilitates ST calling as well as studying the population structure and global epidemiology of bacteria [3].

Apart from the unambiguous nature of MLST, and the ease of exchanging and comparing seven-number allelic profiles, MLST has another advantage. The allelic profiles of isolates can be obtained from clinical material by PCR amplification of the seven house-keeping loci directly from CSF or blood. Thus isolates can be precisely characterized even when they cannot be cultured from clinical material. A practical disadvantage of MLST remains the relatively high cost of sequencing multiple targets. In addition, the discriminatory ability of the present MLST schemes, examining seven housekeeping loci, is suboptimal for several epidemiological questions involving more microepidemiological analysis [5-9]. MLST further requires bioinformatics and genetics expertise in order to properly interpret the data [10].

The recent major advances in DNA sequencing technology, with high-throughput bacterial genome sequencing, allow for the sequencing of the genomes of many thousands of isolates of a pathogen species. Indeed, massively parallel sequencing generates vast numbers (millions) of short sequences, for which the term "reads" is used, which range from about 50 over a few hundred to a few thousand nucleic acids in length.

These short sequences require assembly before most genome analyses can begin. Genomic analytical techniques have traditionally focused entirely on assembly based approaches [11], where the raw data is either aligned against a closely related reference genome [12-13], or de novo assembled and scaffolded using a variety of algorithms [14-16]. The traditional process of aligning these reads to a reference genome is time consuming and the current alignment tools make different compromises between the accuracy and the speed of mapping. It is further computationally challenging to assemble short reads without a reference. Genome assembly remains a very difficult problem, made more cumbersome with shorter reads and unreliable long-range linking information.

Despite the technical difficulties involved in using short read sequencing technologies, the availability of sequence information originating not just from a small part of the genome, but from the entire genome, opens up enormous possibilities for typing bacterial strains. In particular, with the advent of high-throughput sequencing technologies, the restricitive setting of using 7 genes to type bacterial strains, a number which was for a large part inspired by cost and effort, completely disappears, and doing MLST on a whole-genome scale becomes a feasible strategy. In this context, a locus is defined as a set of sequences of nucleic acids or amino acids that are closely related in some sense, for instance, based on sequence similarity, or based on biological or functional grounds, i.e. phenotypic similarity. The nucleic acid or amino acid sequences in a locus are called alleles, or variants for the locus.

To perform MLST, whole-genome MLST, or similar analyses based on short reads, a number of approaches have been developed.

Certain assembly-free approaches in genomic analyses rely on local read mapping, which involves the comparison of each one of the reads with a partial reference genome, thereby obtaining one alignment, or more, between each read and the partial genome. One technique known in the art is Short Read Sequence Typing (SRST), which uses local read mapping and performs all calculations based on this mapping, like allele assignment score [17]. SRST employs the Burrows-Wheeler Aligner (BWA) and the Sequence Alignment/Map format (SamTools) software tools. The SRST algorithm is based on a local assembly, and does not use a k-mer profile approach for allele detection and locus prediction.

Another assembly-free technique is SpolPred, aimed at performing in silico spoligotyping, a typing technique for bacteria in the *Mycobacterium tuberculosis* complex (MTBC), of which *Mycobacterium tuberculosis,* the causative agent of tuberculosis, is the most famous one. Spoligotyping is based on the presence or absence of a set of 43 particular nucleotide sequences (called *spacers*) within the entire genome of a MTBC sample, for which only the presence or absence of the 43 loci matters, and not the precise allelic variant. Since tuberculosis bacterium strains vary in the occurrence of this particular set of spacers, each of these strains produces a distinctive spot pattern (the spot term deriving from hybridization assays, the classical way of determining presence or absence of the spacers), which spot pattern is then translated into a numerical code of 15 digits (known as octal code) for each strain. The web-based database SITVITWEB contains 2740 shared types, also known as spoligotype international types (SITs), found among 58180 clinical isolates, which are grouped into a list of 62 (sub)lineages that are useful for studying the geographic distribution of MTBC (sub)lineages. SpolPred proceeds by using a 25-bp unique spacer (sequence) which is queried against each read allowing up to one mismatch. The appearance of all expected queries is eventually translated into an octal code, which is then matched to a spoligotype in the SITVITWEB database [18]. Unfortunately, SpolPred does not predict the presence of unknown variants.

The software package BIGSDb uses de novo assembly (in particular, the Velvet Optimizer algorithm [39]) to assemble a set of contigs from the short reads, and then uses BLAST [40] to identify the allelic variants present in the sample [19].

From the above description, it is clear that the concept of building a unique k-mer profile for a locus, thus bringing together the characterizing features of a number of alleles (or variants), is to the best knowledge of the inventors, still lacking in the prior art.

## Summary of the invention

An objective of the present invention relates to the provision of a method for (sub-)typing nucleic acid and amino acid sequences, which method is not limited to a particular microorganism.

An objective of the present invention relates to the provision of a method for (sub-)typing nucleic acid and amino acid sequences, which method can be applied to sequences comprising non-natural, modified or analog nucleic acids or amino acids.

An objective of the present invention relates to the provision of a method for (sub-)typing nucleic acid and amino acid sequences that are not assembled. In other words, a method for (sub-)typing short reads.

An objective of the present invention relates to the provision of a method for (sub-)typing nucleic acid and amino acid sequences that are assembled or partially assembled.

An objective of the present invention relates to the provision of a method for (sub-)typing nucleic acid and amino acid sequences that are not assembled, and originate from samples which have not been cultured. In other words, a method for (sub-)typing short reads, which originate from samples which have not been cultured.

An objective of the present invention relates to the provision of a method for (sub-)typing nucleic acid and amino acid sequences that are assembled or partially assembled, and originate from samples which have not been cultured.

An objective of the present invention relates to the provision of a method for detecting the presence of known alleles of a locus in nucleic acid and amino acid sequences that are not assembled. In other words, a method for detecting the presence of known alleles of a locus in short reads.

An objective of the present invention relates to the provision of a method for detecting the presence of known alleles of a locus in nucleic acid and amino acid sequences that are assembled or partially assembled.

An objective of the present invention relates to the provision of a method for detecting the presence of unknown alleles of a locus in nucleic acid and amino acid sequences that are not assembled. In other words, a method for detecting the presence of unknown alleles of a locus in short reads.

An objective of the present invention relates to the provision of a method for detecting the presence of unknown alleles of a locus in nucleic acid and amino acid sequences that are assembled or partially assembled.

An objective of the present invention relates to the provision of a method of (sub-)typing that is not limited to the housekeeping genes, i.e. it can be used to analyse any one or more alleles of any locus.

An objective of the present invention relates to the provision of a method of (sub-)typing nucleic acid and amino acid sequences for more than one locus simultaneously.

An objective of the present invention relates to the provision of a method of (sub-)typing with a higher level of at least one of the key performance criteria selected from reproducibility, typeability, objectivity, portability, discriminatory power, and epidemiological concordance.

The present invention relates to a method for determining the presence or absence of one or more predefined nucleic acid sequences (referred to as alleles) in a set of nucleic acid sequences (referred to as read sequences), said method comprising:

a) defining a k-mer, which is a nucleic acid sequence of a length "k" (for any natural number k), and a k-mer space which consists of all the permutations of nucleic acids ($4^k$) with the selected length "k", and wherein those nucleic acid sequences which are each other's reverse complement (that is, equal apart from their difference in direction, said direction being either forward (5'→3') or reverse (3'→5') direction), are considered as equivalent within the k-mer;

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) providing a set of read sequences;

d) for each one or more predefined alleles and corresponding allele-associated k-mer sets, determining the number of occurrences of each k-mer (of the allele-associated k-mer set) in said set of read sequences, wherein those k-mers which are equivalent in the forward (5'→3') and reverse (3'→5') direction are defined as equivalent k-mers within the set of k-mers, but still can be discernable from each other if desired; wherein an occurrence of a k-mer is revokable based on suitable quality scores for individual nucleic acids in the read sequence;

e) filtering the determined number of occurrences of each k-mer (of the allele-associated k-mer set) from step d); by resetting this number of occurrences of each k-mer to 0 if: i) the total number of occurrences (being either in the forward (5'→3') or reverse (3'→5') direction) is below a predefined threshold, ii) the total number of occurrences in the forward (5'→3') direction is below a predefined threshold, or iii) the total number of occurrences in the reverse (3'→5') direction is below a predefined threshold;

f) determining the presence or absence of each predefined allele in the read sequences based on the filtered number of occurrences of each k-mer (of the allele-associated k-mer set) obtained from step e).

The present invention further relates to a method for determining the presence or absence of one or more sets (referred to as loci) containing one or more predefined nucleic acid sequences (referred to as alleles) in a set of nucleic acid sequences (referred to as read sequences), said method comprising:

a) defining a k-mer, which is a nucleic acid sequence of a length "k" (for any natural number k), and a k-mer space which consists of all the permutations of nucleic acids ($4^k$) with the selected length "k", and wherein those nucleic acid sequences which are each other's reverse complement (that is, equal apart from their difference in direction, said direction being either forward (5'→3') or reverse (3'→5') direction), are considered as equivalent within the k-mer;

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) for each locus, determining which one or more allele-associated k-mer sets are present in a locus, and optionally determining the number of occurrences of each k-mer as defined in step a);

d) providing a set of read sequences;

e) for each locus and associated allele-associated k-mer sets, determining the presence of said one or more allele-associated k-mer sets and the number of occurrences of each k-mer (of each of the allele-associated k-mer sets) in said set of read sequences, wherein those k-mers which are equivalent in the forward (5'→3') and reverse (3'→5') direction are defined as equivalent k-mers within the set of k-mers, but still can be discernable from each other if desired; wherein an occurrence of a k-mer is revokable based on suitable quality scores for individual nucleic acids in the read sequence;

f) filtering the determined number of occurrences of each k-mer (of each of the allele-associated k-mer sets) from step e); by resetting this number of occurrences of each k-mer to 0 if: i) the total number of occurrences (being either in the forward (5'→3') or reverse (3'→5') direction) is below a predefined threshold, ii) the total number of occurrences in the forward (5'→3') direction is below a predefined threshold, or iii) the total number of occurrences in the reverse (3'→5') direction is below a predefined threshold;

g) determining the presence or absence of a loci containing one or more predefined alleles in the read sequences based on the filtered number of occurrences of each k-mer (of each of the allele-associated k-mer sets) obtained from step f).

The present invention further relates to a method for determining the presence or absence of one or more predefined amino acid sequences (referred to as alleles) in a set of amino acid sequences (referred to as read sequences), said method comprising:

a) defining a k-mer, which is an amino acid sequence of a length "k" (for any natural number k), and a k-mer space which consists of all the permutations of amino acids ($20^k$-$22^k$) with the selected length "k";

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) providing a set of read sequences;

d) for each one or more predefined alleles and corresponding allele-associated k-mer sets, determining the number of occurrences of each k-mer (of the allele-associated k-mer set) in said set of read sequences, wherein an occurrence of a k-mer is revokable based on suitable quality scores for individual amino acids in the read sequence;

g) filtering the determined number of occurrences of each k-mer (of the allele-associated k-mer set) from step d); by resetting this number of occurrences of each k-mer to 0 if the total number of occurrences is below a predefined threshold;

h) determining the presence or absence of each predefined allele in the read sequences based on the filtered number of occurrences of each k-mer(of the allele-associated k-mer set) obtained from step e).

The present invention further relates to a method for determining the presence or absence of one or more sets (referred to as loci) containing one or more predefined amino acid sequences (referred to as alleles) in a set of amino acid sequences (referred to as read sequences), said method comprising:

a) defining a k-mer, which is an amino acid sequence of a length "k" (for any natural number k), and a k-mer space which consists of all the permutations of amino acids ($20^k$-$22^k$) with the selected length "k";

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) for each locus, determining which one or more allele-associated k-mer sets are present in a locus, and optionally determining the number of occurrences of each k-mer as defined in step a);

d) providing a set of read sequences;

e) for each locus and associated allele-associated k-mer sets, determining the presence of said one or more allele-associated k-mer sets and the number of occurrences of each k-mer (of each of the allele-associated k-mer sets) in said set of read sequences, wherein an occurrence of a k-mer is revokable based on suitable quality scores for individual amino acids in the read sequence;

f) filtering the determined number of occurrences of each k-mer (of each of the allele-associated k-mer sets) from step e); by resetting this number of occurrences of each k-mer to 0 if the total number of occurrences (being either in the forward (5'→3') or reverse (3'→5') direction) is below a predefined threshold;

g) determining the presence or absence of a loci containing one or more predefined alleles in the read sequences based on the filtered number of occurrences of each k-mer(of each of the allele-associated k-mer sets) obtained from step f).

The present invention further relates to a system for determining the presence or absence of one or more loci containing one or more predefined nucleic acid or amino acid sequences or variants thereof, or for determining the presence or absence of one or more predefined nucleic acid or amino acid sequences or variants thereof, in a set of read sequences, the system comprising at least one processor and an associated storage medium containing a program executable by means of said at least one processor, said system comprising software code portions executing the steps as defined in any one of the embodiments presented herein, in any logical order.

The present invention further relates to a non-transient storage medium on which a computer program product is stored comprising software code portions in a format executable on a computer device and configured for performing the steps as defined in any one of the embodiments presented herein, in any logical order, when executed on said computer device.

The present invention further relates to a computer program product executable on a computer device and comprising software code for executing the method according to any one of the embodiments presented herein, when run on said computer device.

**Brief description of the Figures**

Figure 1 shows a comparison of a portion of the allele sequences found by the algorithms in BIGSDb and the one of the present invention for isolate OXC6347 at the locus CAMP1442. Allele 69 is from the BIGSDb algorithm, and allele 1 is from the algorithm of the present invention.

Figure 2 shows the existing reads comprising position 82126.

Figure 3 depicts the number of discrepant alleles as identified by the BIGSDB algorithm (shown in black) and by the algorithm of the present invention (shown in white).

**Description of the invention**

The present invention relates to a method, a system, a non-transient storage medium, and a computer program product, as defined herein and in the claims.

In one embodiment, in the method according to any one of the embodiments presented herein, in the case of absence of a predefined allele or of a loci containing one or more predefined aleles, the method further comprises determining the percentage of sequence identity of one or more of the k-mer when compared to the read sequences.

In one embodiment, in the method according to any one of the embodiments presented herein, the set of read sequences are unassembled, assembled, or partially assembled sequences.

In one embodiment, in the method according to any one of the embodiments presented herein, the unassembled sequences are obtained from a sequencing platform selected from Sanger sequencing, pyrosequencing, sequencing by synthesis, and any other type that produces sequences of nucleic acids or amino acids.

In one embodiment, in the method according to any one of the embodiments presented herein, the unassembled, assembled, or partially assembled sequences are obtained from any biological material, or in silico data.

In one embodiment, in the method according to any one of the embodiments presented herein, the biological material is selected from one or more organisms and any part thereof.

In one embodiment, in the method according to any one of the embodiments presented herein, the one more organisms is selected from prokariots including bacteria and archaea, from virus, fungi, microscopic arthropods, microscopic crustacean, any pathogenic, chimeric, or artificially-created microorganism, and any mixture thereof.

In one embodiment, in the method according to any one of the embodiments presented herein, the k is selected from 11 to 71 nucleic acids.

In one embodiment, in the method according to any one of the embodiments presented herein, the k is selected from 5 to 23 amino acids.

In one embodiment, in the method according to any one of the embodiments presented herein, the method is used for typing or subtyping; multi-locus sequence typing (MLST); extended multi-locus sequence typing (eMLST); ribosomal multi-locus sequence typing (rMLST); core genome sequence typing (cgMLST); whole genome multi-locus sequence typing (wgMLST, MLST+); spoligotyping for Mycobacterium Tuberculosis; large sequence polymorphism (LSP) detection for Mycobacterium Tuberculosis; Taqman®-based SNP analysis; single-locus sequence typing (or allelic typing); antibiotic resistance typing; antigen serotyping; SPA-typing (Serine Protease Autotransporters of Enterobacteriaceae); HIV, HBV, or HCV drug resistance prediction; DRU (direct-repeat unit) typing; mycobacterial interspersed repetitive units (MIRU) typing, variable number of tandem repeats (VNTR) typing, clustered regularly interspaced short palindromic repeats (CRISPR) typing.

In one embodiment, the sequence reads originate from a sample.

In one embodiment, the sequence files may be in FASTQ format. Both forward and reverse reads are supported.

The term "nucleic acid" refers both to deoxyribonucleic acid (DNA) and ribonucleic acid (RNA).

The term "alleles" refers to variant forms of the same gene, occupying the same locus on homologous chromosomes, and governing the variants in production of the same gene product.

The term "locus" is defined as a set of sequences of nucleic acids or amino acids that are closely related genotypically (based on sequence identity) or phenotypically (based on observable characteristics or traits, such as its morphology, development, biochemical, biological, functional, or physiological properties, phenology, behavior, and products of behavior). The nucleic acid or amino acid sequences in a locus are called alleles or variants for that locus.

A "string" is a sequence of characters.

A "k-mer" is a nucleic acid or amino acid sequence string of length k. Mer refers to the unit of a nucleotide or protein sequence. For a nucleotide sequence, the unit will be a base selected from A, C, T, and G. Modified, analogues, or non-natural bases are also encompassed in the present invention, e.g. and without being limited to, I (Hypoxanthine), X (xanthine), $m^7G$ (7-methylguanine), D (5,6-Dihydrouracil), $m^5C$ (5-Methylcytosine), 5-hydroxymethylcytosine, aminoallyl nucleotide, isoguanine, isocytosine, the fluorescent 2-amino-6-(2-thienyl)purine, pyrrole-2-carbaldehyde, and the like. For an amino acid sequence, the unit will be an amino acid selected from A, R, N, D, C, E, Q, G, H, I, L, K, M, F, P, S, T, W, Y, and V. Modified, analogues, or non-natural amino acids are also encompassed in the present invention, e.g. and without being limited to, U, or O. In addition, the method of the invention can also accommodate those nucleic acid codes (such as K, M, R, Y, S, W, B, V, H, D, X, N, T/U, X/N, , -) or amino acid codes (such as B, Z, J, X), when the sequencing platform cannot conclusively determine the identity of such a residue.

A k-mer is usually a short sequence of nucleic acids or amino acids of length k, or in computer terminology, a string of length k. It can also be understood, for the non-skilled person in the art, as a word of length k. The string or "word" use in a locus defines the characteristics of the same locus, and, to some extent, allows to discriminate one locus from another. The k-mer is preferably long enough to be specific and short enough to fit in the read. The choice of the length k of this short sequence should take into account sequencing errors. As the k-mers of the read will be matched exactly to the allele sequences, the k-mer that is found in the read, should not contain a sequencing error. Therefore, the length of the k-mer should be small enough to fit in between sequencing errors. For instance, supposing that we have a read sequence length of 100 bp and a sequencing error of 1%, then in each read one can expect to find a 50 bp subsequence that does not contain a sequencing error. Therefore, it does not make sense to take k-mers of length larger than 50, because they will very often contain a sequencing error and thus not match with any allele. On the other hand, k-mers that are rather short become less specific, and therefore cause more overlap between k-mer profiles.

In the case of nucleic acids, the k length is preferably between 1 and 251 bases, more preferably between 19 and 65 bases, most preferably about 35 bases long. In the case of amino acids, the k length is preferably between 1 and 81, more preferably between 5 and 41 amino acids, most preferanbly about 9 or 11 amino acids long. 'k's with odd numbers are preferred.

The terms "typing" and "sub-typing" as used herein refer to a molecular biology technique based on DNA sequence analysis that identifies, classifies, and compares organisms and their subtypes.

The term "set of read sequences", usually refers to a plurality of read sequences, i.e. two or more read sequences, but in the case of assembled sequences, said set may consist of only one read sequence.

The sequence analysis strategy of the method of the present invention is based on a gene-by-gene approach. To set up this analysis strategy, a reference framework [43] is first created. Starting from a set of genomes, this is accomplished by taking a number of contiguous regions from each genome (most often coding sequences or genes), ordering them into consistent groups (each of which is called a *locus),* and, for each locus, considering the list of variants that have been found for this locus (each of which is called an *allele)*. Once such a reference framework has been set up, samples are compared based on allelic variation: two samples have a degree of similarity proportional to the number of loci that are present in both samples and that have the same allele for that locus. Therefore, the crucial point of the gene-by-gene analysis strategy is determining for each locus whether it is present in the sample and, if so, in which allelic variant. Given genomic sequence information in any form, the method of the invention i) detects the presence of known variants of a locus in the genomic data, and ii) detects the presence of a locus, in the sense that it predicts the presence of hitherto unknown variants of a locus in the genomic data.

Using the known variants of a locus and the genomic sequence information of a sample, the method of the invention gives an answer to the following questions: i) which of the known variants is/are present in the genomic sequence information of the sample; and ii) which of the loci is/are present in the genomic sequence information of the sample; iii) if none of the known variants is present in the sample, is there an unknown but closely related (in terms of sequence identity) sequence of nucleotides or proteins present in the genomic sequence information of the sample.

For each locus, the method of the invention first calculates a profile of the k-mer occurrence in this locus. A k-mer is a short sequence of nucleotides or proteins of length k, and can be thought of as a word of length k. The word occurrence of a locus defines its characteristics, and, to a large extent, allows to discern one locus from another. For each variant in a locus, a k-mer profile is calculated as well. Again, the word use of an allele defines its characteristics, and, to a large extent, allows to discern one allele from another.

The presence of a k-mer profile for a specific allele in the genomic sequence information of the sample is indicative of the presence of the allele. If no allele-specific k-mer profile can be found, a good representation of the k-mer profile of the entire locus is indicative of the presence of a hitherto unknown allele for this locus.

In one embodiment, in the system according to any one of the embodiments presented herein, said system comprises one or more of the following: a personal computer, a portable computer, a laptop computer, a netbook computer, a tablet computer, a smartphone, a digital still camera, a video camera, a mobile communication device, a personal digital assistant, a scanner, or a multi-function device.

In one embodiment, in the non-transient storage medium or computer program product according to any one of the embodiments presented herein, the computer device is selected from a personal computer, a portable computer, a laptop computer, a netbook computer, a tablet computer, a smartphone, a digital still camera, a video camera, a mobile communication device, a personal digital assistant, a scanner, and a multi-function device.

Description of the algorithm

*Definition of Alphabets, words, k-mer space and k-mer profiles*

Let $\Sigma$ be an *alphabet* consisting of s letters. The alphabets we are primarily concerned with (but not restricted to) are:

the DNA *nucleotide alphabet*

$$\Sigma_{\mathrm{NT}} = \{A, C, G, T\},$$

the RNA *nucleotide alphabet*

$$\Sigma_{\mathrm{NT}} = \{A, C, G, U\},$$

and the *amino acid alphabet*

$$\Sigma_{\mathrm{AA}} = \{G, P, A, V, L, I, M, C, F, Y, W, H, K, R, Q, N, E, D, S, T\}.$$

A *word w* on an alphabet $\Sigma$ is a sequence of letters from the alphabet $\Sigma$. For any $k \in \mathbb{N}$, a *k-mer is* a word of size $k$. The set of all k-mers on the alphabet $\Sigma$ is called the *k-mer set,* and consists of $k^s$ words.

If we define the length of a mer string to be for instance k = 2, then in the case of DNA nucleotide sequences, and since DNA has 4 different types of bases (A,C,T,G), a mer of k=2, will result in the following possible combinations ($4^k = 4^2$):

AA, AC, AG, AT, CA, CC, CG, CT, GA, GC, GG, GT, TA, TC, TG, TT.

When working in the *k-mer set* based on the nucleotide alphabet $\Sigma_{\mathrm{NT}}$, we have to consider forward and reverse sequences, since some of the combinations are equivalent to others: for example AC (in 5'$\to$3' direction) is equivalent to GT (in 3'$\to$5' direction).

Therefore, for a mer of k=2, the number of possible combinations is reduced to the following:

```
AA / TT
AC / GT
AG / CT
   AT
CA / TG
CC / GG
   CG
GA / TC
   GC
   TA
```

It is therefore useful to look at the quotient space under the equivalence relation defined by reverse complementing. The *reverse complement* of a word on the nucleotide alphabet $\Sigma_{NT}$ is the reverse (so back-to-front) of the original word, in which every letter is replaced by its complement, that is,

$$A \rightarrow T, C \rightarrow G, G \rightarrow C, T \rightarrow A.$$

As an example, the reverse complement of the word *ACAGTCA* is *TGACTGT*. One can think of the quotient space in terms of its representatives (one for each equivalence class). A straightforward way of picking a representative for each equivalence class would be to take the lexicographically smallest word in each class. In the example, *ACAGTCA* would be the representative for the class {*ACAGTCA, TGACTGT*}.

The reason for considering this equivalence relation is that the input data from next-generation sequencers most often is not oriented, in the sense that the same genomic location might be read as one particular word or its reverse complement. This is obviously not the case with amino-acid sequences. Therefore, in the remainder of this text the term *k*-mer set will be used to denote either the actual *k*-mer set on the alphabet in question, or, in case the alphabet is the nucleotide alphabet, the quotient space of the actual k-mer set under the reverse complementing equivalence relation.

The *k-mer space* for the alphabet $\Sigma$ is the multidimensional real vector space that has a one dimension for each *k*-mer in the *k*-mer set.

Let w be a word on an alphabet $\Sigma$ of length at least *k*. The *k*-mer profile of the word *w* is a vector in *k*-mer space, where each number in the vector indicates how many times the *k*-mer corresponding to that dimension has been used in w. For instance, the word *ACAGTCA* on the alphabet $\Sigma_{NT}$ has the following 2-mer profile:

| AA,TT | AC,GT | AG,CT | AT | CA,TG | CC,GG | CG | GA,TC | GC | TA |
|-------|-------|-------|----|-------|-------|----|-------|----|----|
| 0 | 2 | 1 | 0 | 2 | 0 | 0 | 1 | 0 | 0 |

*Definition of Gene-by-gene systems, loci and alleles*

In the context of gene-by-gene systems, the generic terminology introduced above is often completed with a number of synonyms and additional concepts.

Let $\Sigma$ be an alphabet. An *allele* $\alpha$ on the alphabet $\Sigma$ is a word on the alphabet $\Sigma$. For instance, allele

*TTAGAGCGCGCTGATATCGGTATTGACGCTAAAGCCGCGATCGAGGCTGACGCT*
*GTTGCCCGCCGCGTCGCTCACGACTGCCGTTACGGTGTAAGTGCCGTTGGTAAG*
*GCCTGCAAGGGTGTCGGCAGGCAGATTGACTTCCCAGCGGCCTGCGGAATCCAC*

is an allele originating from a nucleotide-based gene-by-gene system for the bacterial genus *Cronobacter.*

A *locus l* is a set of alleles, thus variants of the above sequence:

$$l = \{a_1, a_2, \dots, a_n\}$$

For instance, the locus *l* corresponding to the gene *ycbC* bacterial genus *Cronobacter*consists of 15 alleles,

$$l = \begin{cases} TCACTTCTGCCCTGGGTCGCCTGAGCCCACGCCTTTTACC \ldots , \\ TCACTTCTGCCCTGGGTCGCCTGAGCCCACGCCTTTTACC \ldots , \\ \ldots , \\ TCACTCCTTCCCTGGGTCGCCTGATGCCACGCCTTTCATC \ldots \end{cases}$$

Only the first, second and 15th allele are shown here, and for each allele only the first 40 bases are included.

A *gene-by-gene system* is a set of loci,

$$L = \{ l_1, l_2, \ldots, l_n \}.$$

For instance, for the bacterial genus *Cronobacter,* there is a gene-by-gene system consisting of 11,168 loci.

As can be seen from the above example, a locus usually does not bring together a random set of alleles. There are criteria that determine which alleles are put in the same locus, and which loci are included in a gene-by-gene system. For instance, one criterion including an allele in a locus could be the minimal pairwise word distance between the candidate allele and the known alleles already present in the locus, taking into account an optimal alignment of each known allele and the candidate allele. In general, these criteria are based on biological grounds.

The input data for the algorithm is i) a gene-by-gene system (given by its set of loci, and thus its set of alleles) on an alphabet $\Sigma$; and ii) a sample, given by a set of words on the same alphabet $\Sigma$.

*Step 1: Building up the reference data for each allele and locus*

For each of the alleles in each of the loci in the gene-by-gene system, a *k*-mer profile is calculated. For each allele, the set of *k*-mers used in that allele are retained. Explained otherwise, we build an allele/k-mer incidence matrix. We record for example for allele 1, whether it contains each k-mer out of the list of all possible combinations of k-mers.

A C A G T C A

Table 1

| AA | 0 |
|----|---|
| AC | I |
| AG | I |
| AT | 0 |
| CA | II |
| CC | 0 |
| CG | 0 |
| CT | 0 |
| GA | 0 |
| GC | 0 |
| GG | 0 |

Table 2

| AA / TT | 0 |
|---------|---|
| AC / GT | 2 |
| AG / CT | 1 |
| AT | 0 |
| CA / TG | 2 |
| CC / GG | 0 |
| CG | 0 |
| GA / TC | 1 |

Table 3

| AC / GT | 2 |
|---------|---|
| AG / CT | 1 |
| CA / TG | 2 |
| GA / TC | 1 |

| GT | I |
|---|---|
| TA | 0 |
| TC | I |
| TG | 0 |
| TT | 0 |

| GC | 0 |
|---|---|
| TA | 0 |

Table 1 shows the number of occurrences of each of the 16 '2-mer' for allele 1. Table 2 takes into account the equivalent 2-mer in the forward and reverse directions. In the incidence matrix, the matrix element (k-mer,allele) is a number (1,2, ...) if the allele contains that given number of times the specific k-mer, and is 0 otherwise. Since a $(4^2/2)$ x will obtain too many results, too many to fit in a computer's memory, and much of the results are 0, a more performant representation is to record only the things that do occur. Therefore we reduce it to the matrix of Table 3 above.

Table 3 represents the incidence matrix which shows the number of occurrences of each k-mer in allele 1 (the k-mer that never occur are removed to save computer memory).

Since one locus has different known alleles (variants), we create the incidence matrix for each known allele.

We obtain a vector of strings and numbers for each allele. For example, for alleles 1-3 of Locus 1, the vector will have the following expression (the vector can be built for as many alleles and loci as desired):

| Locus 1 | Allele 1 ACAGTCA | AC/GT: 2  AG/CT:1  CA/TG:2  GA/TC:1 |
|---|---|---|
| | Allele 2 ACAGTCC | AC/GT: 2  AG/CT:1  CA/TG:1  CC/GG:1  GA/TC:1 |
| | Allele 3 ACAGTCT | AC/GT: 2  AG/CT:2  CA/TG:1  GA/TC:1 |
| Locus 2 | Allele 1 | ... |
| | Allele 2 | ... |
| | Allele 3 | ... |
| | Allele 4 | ... |
| Locus 3 | Allele 1 | ... |
| | Allele 2 | ... |
| ... | | |

*Step 2: Screening the sample data (reads) against the reference k-mer sets*

For each of the *k*-mers used in each of the alleles, it is determined how many times this *k*-mer appears in the words of the sample data, i.e. the reads. If the sample data not only contains words but also individual quality scores for each letter in a word, only high-quality *k*-mers in the reads are being used. In particular, *k*-mers can be discarded if there are too many low-quality letters within one *k*-mer. An example of base quality scores, and without being limited to, are the PHRED scores, which indicate the probability that the base call is wrong. For instance, a quality score of 20 indicates that the probability that the base call is wrong, is 1 / 100. In other words, if you have 100 bases with quality 20 then you can expect one of them to be wrong. That being said, these quality scores can be used to decide wether a k-mer is thrustworthy or not. Using a minimum quality threshold (say 20) and a maximum number of violations threshold (say 3), a k-mer is accepted if it contains at most 3 bases that have a quality below 20.

Usually, determining the presence or absence of the allele-associated k-mer set within the read sequences can be performed by scanning each of these read sequences with the k-mer set. In practice, in a set of nucleotide sequences, also named as reads, which reads are unassembled, but can also be assembled in one single sequence, or partially assembled in some sequences, we pose for allele 1 (ACAGTCA) a query built with the Boolean operator 'AND': AC/GT: 2 AND AG/CT :1 AND CA/TG: 2 AND GA/TC: 1

The software will run the query for each read where sequences are compared using exact k-mer matching. If the k-mer set is retrieved within the read sequences, it is scored as true and counted and the one or more matching alleles of the one or more loci are presented.

| Allele 1 | AC/GT: 2 | AG/CT:1 | CA/TG: 2 | GA/TC:1 |
|---|---|---|---|---|
| | | | | |
| reads | AC/GT:69 | AG/CT:34 | CA/TG:60 | GA/TC:33 |

*Step 3: Filtering*

Quality control measures are incorporated in the method according to the invention in order not to consider an unknown allele in cases which are due to a poor sequence quality, which is likely to result in errors in sequencing.

The *k*-mers are filtered on the number of occurrences in the entire sample. In particular, all *k*-mers that appear less than a predefined threshold, are discarded. When using the nucleotide alphabet, one can also discard equivalence classes of *k*-mers for which not all equivalent *k*-mers are present in the sample data. In other words, a k-mer (or, in this case, an equivalence class containing at most 2 k-mers) is considered as present if and only if all of its representatives can be found in the reads. This means that each k-mer must be seen in the forward and in the reverse direction. This trick is used to avoid sequencing errors that appear only on one strand. In the case of such an error, you would get a wrong k-mer in one direction, but never see it in the other direction, and therefore discard it.

The filtering step in the method acording to the present invention is basically a quality control step and may take many forms selected from:

calculating the coverage, which is the number of times the same k-mer has been scanned, wherein the k-mer is retained if its coverage is higher than a predefined threshold (which is by default set to 3, but depends on the type of input data, whether it be short reads or assembled or partially assembled sequences),
calculating the coverage of the individual representatives of a k-mer's equivalence class, wherein the k-mer is retained if the coverage of all the individual representatives is higher than a predefined threshold (which is by default set to 1, but depends on the type of input data, whether it be short reads or assembled or partially assembled sequences).

*Step 4: Allele detection*

An allele is predicted as present in the sample data if and only if (iff) all k-mers in the k-mer set for that allele can be found in the sample data (after filtering).

If the k-mer set is not retrieved within the read sequences, it is scored as false and provides those short reads with a sequence similarity of at least X% to the queried allele, and presents them as an unknown allele related to a known allele.

In particular, the following questions are answered:

Are all the specific combination of "words" (k-mer) of allele 1 present in the reads, and with a similar number of occurrences?

If yes, the allele 1 is present.

If no, the next question is: which are not matching? For example:

| | Allele 1 | A C A G T C A | | | |
|---|---|---|---|---|---|
| | read | | A C A G T C G | | |
| Allele 1 | AC/GT: 2 | AG/CT:1 | CA/TG: 2 | GA/TC:1 1 | |
| | | | | | |
| read | AC/GT:68 | AG/CT:34 | CA/TG:30 | CG: 29 | GA/TC:33 |

whereas the 4 k-mers of Allele 1 are present in the read, said read has in addition a fifth k-mer CG, and the k-mer CA/TG is present in a proportion of the half that is present in the Allele 1. This indicates that we are probably confronted with an unknown allele, which in turn allows us to predict a new allele because of the sequence similarity.

*Step 5: Locus presence*

A locus is predicted as present in the sample data if and only if (iff) for at least one of its alleles, the number of *k*-mers in the *k*-mer set for that allele that can be found in the sample data (after filtering), exceeds a predefined threshold.

Similarly, for determining the presence or absence of one or more loci containing one or more alleles in a set of reads, which may be unassembled, assembled in one single sequence, or partially assembled in some sequences, we list all the strings matching for each allele with Boolean operator 'AND', and we list all of the strings set matching each of the alleles of one locus with Boolean operator 'OR'; thereby obtaining a k-mer profile, or an allele-associated k-mer set per locus.

We pose for example for locus 1, containing alleles 1 (ACAGTCA), 2 (ACAGTCC), and 3 (ACAGTCT), a query built with the Boolean operators 'AND' and 'OR', as follows:

AC/GT: 2 AND AG/CT :1 AND CA/TG: 2 AND GA/TC: 1

OR

AC/GT: 2 AND AG/CT:1 AND CA/TG:1 AND CC/GG:1 AND GA/TC:1 OR

AC/GT: 2 AND AG/CT:2 AND CA/TG:1 AND GA/TC:1

The software will run the query for each read where sequences are compared with the queried k-mer profile for loci 1.

Applications

The method of the invention can be applied to (sub-)type any kind of organism, whether it be prokaryotic or eukaryotic. In the literature, several successful attempts at applying a gene-by-gene strategy for the analysis and comparison of biological samples for a broad range of organisms have been reported [25,26,27]. Moreover, but on a smaller scale and using first-generation Sanger sequencing, the gene-by-gene methodology has been used for many years in multi-locus sequence typing for bacteria [23,24].

The method of the invention can be applied to samples that contain only one biological specimen (called *cultured samples*) or to samples that contain many different biological specimens (called *uncultured samples*). In the former case, the next-generation sequencing data obtained from such a sample is usually called whole genome sequencing (WGS) data, in the latter case the next-generation sequencing data obtained from such a sample is usually called shotgun metagenomics data. In addition, the method of the invention can also be applied to expression sequencing data such as RNA-seq or ChipSeq.

The method of the invention can be applied to the analysis of sequence data of any type and of any origin. The sequence data type can be nucleotide or amino acid type sequence data. The origin of the data can be from so-called but is not limited to i) *first-generation* sequencing technologies (e.g. Sanger sequencing); ii) *next-generation* sequencing technologies (e.g. Illumina's GenomeAnalyzer, HiSEQ, MiSEQ, NextSEQ, IoTorrent's PGM, Pacific Biosciences); iii) *third-generation* sequencing technologies (e.g. Oxford NanoPore); or iv) any other present or future technology that provides nucleotide or amino acid sequences.

In one embodiment, the sequence reads may be obtained from whole genome sequencing data. Reads are available over the internet from databases like SRA (Short Read Archive) owned by NCBI (National Center for Biotechnology Information), ENA (European Nucleotide Archive) owned by EBI-EMBL (The European Bioinformatics Institute, Part of the European Molecular Biology Laboratory), DDBJ Sequence Read Archive (DNA Data Bank of Japan).

There are also websites providing MLST information like http://www.mlst.net and http://pubmlst. org, hosting databases for different pathogen species, and there are now additional sites for other species, for example, at University College Cork (http://mlst.ucc.ie) and the Pasteur Institute(http://www.pasteur.fr/mlst). These websites have tools that allow the databases to be queried and the relationships between new isolates and those in the database to be visualized. The relatedness among isolates characterized by MLST and other molecular typing methods has normally been visualized by clustering approaches using, in the case of MLST, the differences in the allelic profiles of isolates, so that those most similar to a query isolate can be identified on a tree.

The typing methods of the present invention can be used for a variety of purposes, for example to understand phylogeny (evolution) and bacterial population genetics, to identify specific strains spreading globally in specific populations and/or in core groups, to identify temporal and geographic changes in strain types as well as the emergence and transmission of individual strains, to establish strain identity/difference in contact tracing or test of cure, to confirm/disprove treatment failures, to resolve medico-legal issues such as sexual abuse, to confirm presumed epidemiological connections or discriminate isolates of suspected clusters and outbreaks. Strain typing coupled with antimicrobial susceptibility data helps in a better understanding of the transmission of specific antibiotic-resistant strains. Ultimately, such information can be applied to design different public health preventive measures and interventions.

The typing methods of the present invention can be used for precise and reliable studies dealing with the macroepidemiology (long-term and global epidemiology) of infections caused by microorganisms, population dynamics over many years or decades, and phylogeny (evolution).

The methods according to the present invention may be used in any single- or multi-locus sequence typing scheme that uses untargeted genomic sequence information (targeted genomic sequence information is by design already associated to a specific locus, and thus needs no further locus or allele detection).

In particular, the method of the invention may be used in, but is in no way restricted to, multi-locus sequence typing for any bacterial organism including traditional multi-locus sequence typing (MLST) as described by Maiden et al. [23]; extended multi-locus sequence typing (eMLST) as described by Didelot et al. [24]; ribosomal multi-locus sequence typing (rMLST) as described by Jolley et al. [25]; whole genome multi-locus sequence typing (wgMLST, MLST+) as described by Cody et al. [26] or Jolley et al. [27]; cgMLST for MTBC [41]; cgMLST for MRSA [42]; and any other typing scheme that is built according to the principles in the publications cited above.

The method of the invention may also be used for the detection of the presence/absence of specific sequences of nucleotides or proteins, such as spoligotyping for Mycobacterium Tuberculosis as described by Groenen et al. [28] or Kamerbeek et al. [29]; large sequence polymorphism (LSP) detection for Mycobacterium Tuberculosis as described by Gagneux et al. [30], Kato-Maeda et al. [31], Tsolaki et al. [32], Hirsh et al. [33], or Fleischmann et al. [34].

The method of the invention may also be used for the detection of single-base variants whose location on the genome is defined by its flanking regions, such as Taqman®-based SNP analysis.

The method of the invention may also be used for single-locus sequence typing (or allelic typing) including for example, rifampicin resistance: rpoA, rpoB (Myco) (cfr. Miller et al. [35] or Telenti et al. [36]); M-protein typing: emm (Spyo) (cfr. Kaufhold et al. [37]); flab (Camp); spa (Saur); slpAst (Cdif); adhesion typing: FimH, and the like.

The method of the invention may also be used for antigen gene sequence typing (AGST) (cfr. Colles and Maiden [38]); antibiotic resistance typing e.g. Tellurium res (ter); antigen serotyping; SPATEs typing (Serine Protease Autotransporters of Enterobacteriaceae) (Ecoli, Shig); HIV, HBV, HCV, drug resistance prediction, and the like.

Further uses include the microepidemiological analysis of strains, which examines the identity of isolates collected during short time periods (days) to a limited number of months or even to a maximum period of a few years. This approach includes typing strains in the following instances: community epidemics; strains in an entire population over a limited time; strains from core groups, larger core groups, or sexual networks; identifying the emergence and transmission of individual (e.g., antimicrobial-resistant) strains; confirmation or discrimination of presumed epidemiological connections in suspected clusters of infection; contact tracing, test of cure, and resolution of medico-legal cases; and characterization of bacterial clones.

The following examples are intended to illustrate the present invention in further detail and should not be interpreted as limiting it thereto.

**Examples**

**Example 1: Allele prediction in *Campylobacter jejuni* sequences**

We used a publicly available gene-by-gene system for the bacterial species *Campylobacter jejuni* [26], and a publicly available sample set of 36 samples. This allowed us to compare the results obtained from the algorithm of the invention with the publicly available results.

Table 1. Samples used in the example. The isolate IDs refer to the publicly available BIGSDb isolate database, the sequence data can be found on the European Nucelotide Archive (ENA).

| Isolate ID | ENA accession | Isolate ID | ENA accession | Isolate ID | ENA accession |
|---|---|---|---|---|---|
| OXC6347 | ERR083963 | OXC6461 | ERR084072 | OXC6564 | ERR108328 |
| OXC6407 | ERR084021 | OXC6632 | ERR108394 | OXC6531 | ERR084142 |
| OXC6592 | ERR108356 | OXC6524 | ERR084135 | OXC6266 | ERR083883 |
| OXC6487 | ERR084098 | OXC6543 | ERR084154 | OXC6598 | ERR108362 |
| OXC6600 | ERR108364 | OXC6615 | ERR108378 | OXC6286 | ERR083902 |
| OXC6449 | ERR084061 | OXC6285 | ERR083901 | OXC6448 | ERR084060 |
| OXC6636 | ERR108398 | OXC6331 | ERR083947 | OXC6520 | ERR084131 |

(continued)

| Isolate ID | ENA accession | Isolate ID | ENA accession | Isolate ID | ENA accession |
|---|---|---|---|---|---|
| OXC6571 | ERR108335 | OXC6459 | ERR084070 | OXC6423 | ERR084037 |
| OXC6565 | ERR108329 | OXC6275 | ERR083892 | OXC6567 | ERR108331 |
| OXC6590 | ERR108354 | OXC6530 | ERR084141 | OXC6457 | ERR084068 |
| OXC6393 | ERR084009 | OXC6251 | ERR083868 | OXC6574 | ERR108338 |
| OXC6527 | ERR084138 | OXC6604 | ERR108368 | OXC6542 | ERR084153 |

Allele prediction was performed with the algorithm of the present invention, and took about 1 minute per sample on standard hardware, whereas the de novo procedure used in BIGSDb took about 15 to 20 minutes on the same hardware. For the 36 samples and the 58259 alleles that had been found in those samples, we found 2 out of 58259 uniquely called loci ('uniquely' referring to that for each of the 58259 loci, one single allele was found) (or about 0.003%) that were different between our results and the published allele designation. The differences in allele designations can be traced back to ambiguous positions, which had been resolved in the de novo assembly BIGSDb process in an idiosyncratic manner.

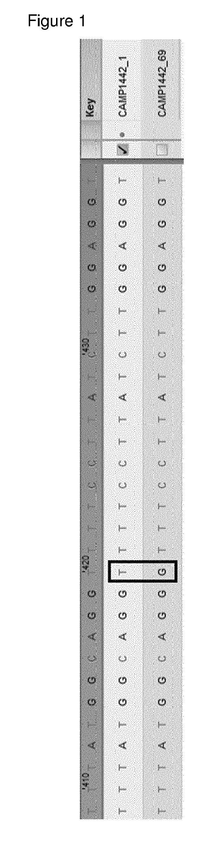

Looking at isolate OXC6347, and, in particular, at the locus CAMP1442, we observed that it had been called allele 69 by BIGSDb, and allele 1 by our algorithm. When looking at allele 69 versus allele 1, we observed that there was a single base difference between these two (T for allele 1 and G for allele 69); please refer to Figure 1.

When we then looked at the de novo assembled sequence around position 82126, and the BLAST alignment of allele 69 (on the left) and allele 1 (on the right),

### Allele 69 – BIGSDb                     Allele 1 - present invention

```
82115  TTTATGGCAGGGTTTCCTTATCTTGGA    82115  TTTATGGCAGGGTTTCCTTATCTTGGA
       |||||||||||||||||||||||||||           |||||||||||  ||||||||||||
409    TTTATGGCAGGGTTTCCTTATCTTGGA    409    TTTATGGCAGGTTTTCCTTATCTTGGA
```

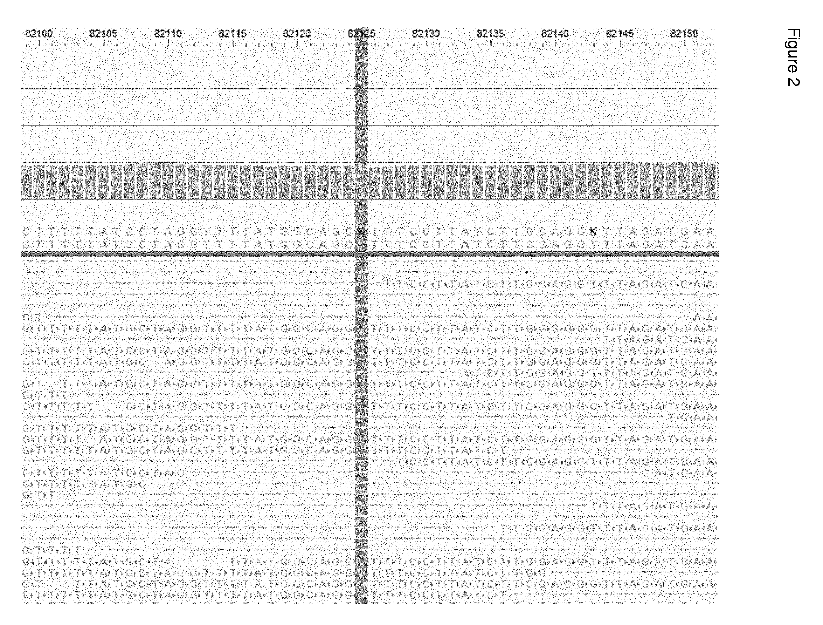

we saw a complete match with allele 69 and a single mismatch with allele 1. This indicated that the allele found in the BIGSDb de novo assembled genome was effectively the allele 69, and not 1. However, when we then mapped the reads back to the de novo assembled genome, and we looked at the same position 82126, we saw that this was an ambiguous position, some reads had G and other reads had T as base; please confer to Figure 2. In particular, we had a coverage of 85, of which 28 reads had a G, and 57 reads had a T. Moreover, all 28 reads having a G were mapped in the forward direction, whereas the reads having a T were mapped in both directions (24 forward and 33 reverse). We also noted that there was another ambiguous base at position 82144 (coverage 94, having 27 G's and 67 T's, all G's were in forward direction, whereas the T's were spread over forward and reverse). When looking at the reads that spanned these two ambiguous positions,

| | | |
|---|---|---|
| G...G | 16 | unknown allele |
| G...T | 1 | allele 69 |
| T...G | 11 | unknown allele |
| T...T | 37 | allele 1 |

we saw that all combinations had more than 10 reads, except for the combination G...T, which only occured once. As the algorithm's minimum coverage parameters had been set to 3 for the total coverage and to 1 for both forward and reverse coverage, the allele 69 had not been picked up, but allele 1 had been picked up because it was the only known allele that was supported both in the forward and the reverse direction.

Whereas both the BIGSDb method and the method of the present invention (with a k-mer based approach) operate at very high accuracy, this example shows that there is a risk associated with the use of de novo assembly without post-assembly base calling correction. As the algorithm of the present invention goes directly back to the reads, there is no such confusion.

**Example 2: Reproducibility and within-patient variation**

We will now show that the algorithm of the present invention provides improved accuracy and sensitivity with respect to the de novo assembly based method. To this end, we again used the publicly available gene-by-gene system for the bacterial species *Campylobacter jejuni* [26], and two publicly available sample sets, the first of which containing 10 samples that had been obtained from the patient once but sequenced twice, and the second containing 17 samples that had been obtained from the patient twice, and also sequenced twice.

In the 10 *Campylobacter* isolates that had been isolated once but sequenced twice, the algorithm of the present invention found no differences between them (except for coverage effects). The *de novo* assembly procedure did show some variation (1 to 7 loci), in particular for paralogous genes. This was caused by small almost random choices made by the assembly algorithm in repeated or almost repeated regions, and the lack of verification that this region was properly assembled.

The biological variation in *Campylobacter jejuni* isolates from the same patient ranged between 0 to 10 alleles different as identified by the algorithm of the invention, and between 0 to 13 alleles different as identified by the BIGSDb algorithm. On average, the variation was 0,07% for the data from our algorithm, compared to 0,22% for BIGSDb data.

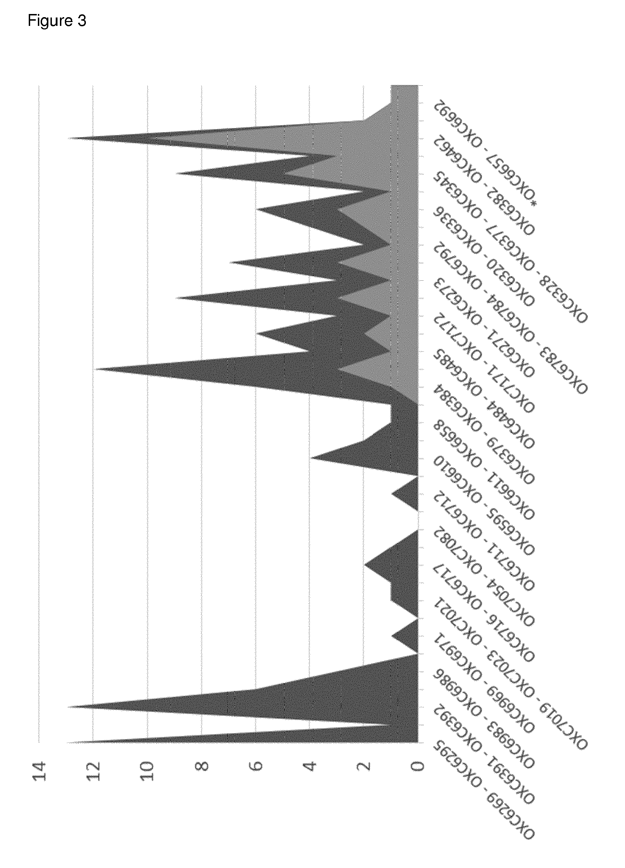

In Figure 3, there is depicted the number of discrepant alleles as identified by BIGSDB (black) and by the algorithm of the present invention (white).

The reason for this more than 3-fold decrease in number of discrepant alleles was mainly caused by almost random choices made by the assembly algorithm in repeated or almost repeated regions, and the lack of verification that this region was properly assembled.

List of journal references

1. Cohen SH, Tang YJ, Silva J Jr. Molecular typing methods for the epidemiological identification of Clostridium difficile strains. Expert Rev Mol Diagn. 2001;1(1):61-70.

2. Spratt BG. The 2011 Garrod Lecture: From penicillin-binding proteins to molecular epidemiology. J Antimicrob Chemother 2012; 67: 1578-1588

3. Knetsch CW, Lawley TD, Hensgens MP, Corver J, Wilcox MW, Kuijper EJ. Current application and future perspectives of molecular typing methods to study Clostridium difficile infections. Euro Surveill. 2013;18(4):pii=20381.

4. Voth DE, Ballard JD. Clostridium difficile toxins: mechanism of action and role in disease. Clin Microbiol Rev. 2005;18(2):247-63.

5. Ison, C. A., et al. 2003. International comparison of molecular typing methods for Neisseria gonorrhoeae, abstr. 364. Abstr. 15th Int. Soc. Sex. Transm. Dis. Res. Congr., Ottawa, Canada.

6. Pérez-Losada, M., K. A. Crandall, J. Zenilman, and R. P. Viscidi. 2007. Temporal trends in gonococcal population genetics in a high prevalence urban community. Infect. Genet. Evol. 7:271-278.

7. Pérez-Losada, M., et al. 2007. Distinguishing importation from diversification of quinolone-resistant Neisseria gonorrhoeae by molecular evolutionary analysis. BMC. Evol. Biol. 7:84.

8. Pérez-Losada, M., R. P. Viscidi, J. C. Demma, J. Zenilman, and K. A. Crandall. 2005. Population genetics of Neisseria gonorrhoeae in a highprevalence community using a hypervariable outer membrane porB and 13 slowly evolving housekeeping genes. Mol. Biol. Evol. 22:1887-1902

9. Tazi, L., et al. 2010. Population dynamics of Neisseria gonorrhoeae in Shanghai, China: a comparative study. BMC Infect. Dis. 10:13.

10. Magnus Unemo and Jo-Anne R. Dillon. Review and International Recommendation of Methods for Typing Neisseria gonorrhoeae Isolates and Their Implications for Improved Knowledge of Gonococcal Epidemiology, Treatment, and Biology. Clinical Microbiology Reviews, July 2011, p. 447-458

11. Pop & Salzberg 2008

12. Li et al. 2008

13. Langmead et al. 2009

14. Warren et al. 2007

15. Zerbino & Birney 2008

16. Simpson et al. 2009

17. Inouye M, Conway T , Zobel J, and Holt KE. Short read sequence typing (SRST): multi-locus sequence types from short reads. BMC Genomics 2012, 13:338

18. Coll F, Mallard K, Preston M, Bentley S, Parkhill J, McNerney R, Martin N, and Clark T. SpolPred: rapid and accurate prediction of Mycobacterium tuberculosis spoligotypes from short genomic sequences. Bioinformatics. 2012 November 15; 28(22): 2991-2993.

19. Jolley K.A., Maiden M.C., BIGSdb: Scalable analysis of bacterial genome variation at the population level, BMC Bioinformatics. 2010 Dec 10;11:595. doi: 10.1186/1471-2105-11-595.

20. Hunter PR, Gaston MA. Numerical index of the discriminatory ability of typing systems: an application of Simpson's index of diversity. J Clin Microbiol. 1988;26(11):2465-6.

21. van Belkum A, Tassios PT, Dijkshoorn L, Haeggman S, Cookson B, Fry NK, et al. Guidelines for the validation and application of typing methods for use in bacterial epidemiology. Clin Microbiol Infect. 2007;13 Suppl 3:1-46.

22. Nadon CA, Trees E, Ng LK, Møller Nielsen E, Reimer A, Maxwell N, Kubota KA, Gerner-Smidt P, the MLVA Harmonization Working Group. Development and application of MLVA methods as a tool for interlaboratory surveillance. Euro Surveill. 2013;18(35):pii=20565.

23. Maiden, M.C.J., Bygraves, J.A., Feil, E., Morelli, G., Russell, J.E., Urwin, R., Zhang, Q., Zhou, J., Zurth, K., Caugant, D.A., et al. (1998). Multilocus sequence typing: A portable approach to the identification of clones within populations of pathogenic microorganisms. Proc. Natl. Acad. Sci. U. S. A. 95, 3140-3145.

24. Didelot, X., Urwin, R., Maiden, M.C.J., and Falush, D. (2009). Genealogical typing of Neisseria meningitidis. Microbiology 155, 3176-3186.

25. Jolley, K.A., Bliss, C.M., Bennett, J.S., Bratcher, H.B., Brehony, C., Colles, F.M., Wimalarathna, H., Harrison, O.B., Sheppard, S.K., Cody, A.J., et al. (2012a). Ribosomal multilocus sequence typing: universal characterization of bacteria from domain to strain. Microbiol. Read. Engl. 158, 1005-1015.

26. Cody, A.J., McCarthy, N.D., Rensburg, M.J. van, Isinkaye, T., Bentley, S., Parkhill, J., Dingle, K.E., Bowler, I.C.J.W., Jolley, K.A., and Maiden, M.C.J. (2013). Real-time genomic epidemiology of human Campylobacter isolates using whole genome multilocus sequence typing. J. Clin. Microbiol.

27. Jolley, K.A., Hill, D.M.C., Bratcher, H.B., Harrison, O.B., Feavers, I.M., Parkhill, J., and Maiden, M.C.J. (2012b). Resolution of a Meningococcal Disease Outbreak from Whole-Genome Sequence Data with Rapid Web-Based Analysis Methods. J. Clin. Microbiol. 50, 3046-3053.

28. Groenen, P.M.A., Bunschoten, A.E., Soolingen, D. van, and Errtbden, J.D.A. van (1993). Nature of DNA polymorphism in the direct repeat cluster of Mycobacterium tuberculosis; application for strain differentiation by a novel typing method. Mol. Microbiol. 10, 1057-1065.

29. Kamerbeek, J., Schouls, L., Kolk, A., Agterveld, M. van, Soolingen, D. van, Kuijper, S., Bunschoten, A., Molhuizen, H., Shaw, R., Goyal, M., et al. (1997). Simultaneous detection and strain differentiation of Mycobacterium tuberculosis for diagnosis and epidemiology. J. Clin. Microbiol. 35, 907-914

30. Gagneux, S., DeRiemer, K., Van, T., Kato-Maeda, M., de Jong, B.C., Narayanan, S., Nicol, M., Niemann, S., Kremer, K., Gutierrez, M.C., et al. (2006). Variable host-pathogen compatibility in Mycobacterium tuberculosis. Proc. Natl. Acad. Sci. U. S. A. 103, 2869-2873.

31. Kato-Maeda, M., Rhee, J.T., Gingeras, T.R., Salamon, H., Drenkow, J., Smittipat, N., and Small, P.M. (2001). Comparing Genomes within the Species Mycobacterium tuberculosis. Genome Res. 11, 547-554.

32. Tsolaki, A.G., Hirsh, A.E., DeRiemer, K., Enciso, J.A., Wong, M.Z., Hannan, M., Salmoniere, Y.-O.L.G. de la, Aman, K., Kato-Maeda, M., and Small, P.M. (2004). Functional and evolutionary genomics of Mycobacterium tuberculosis: Insights from genomic deletions in 100 strains. Proc. Natl. Acad. Sci. U. S. A. 101, 4865-4870

33. Hirsh, A.E., Tsolaki, A.G., DeRiemer, K., Feldman, M.W., and Small, P.M. (2004). Stable association between strains of Mycobacterium tuberculosis and their human host populations. Proc. Natl. Acad. Sci. U. S. A. 101, 4871-4876.

34. Fleischmann, R.D., Alland, D., Eisen, J.A., Carpenter, L., White, O., Peterson, J., DeBoy, R., Dodson, R., Gwinn, M., Haft, D., et al. (2002). Whole-Genome Comparison of Mycobacterium tuberculosis Clinical and Laboratory Strains. J. Bacteriol. 184, 5479-5490.

35. Miller, L.P., Crawford, J.T., and Shinnick, T.M. (1994). The rpoB gene of Mycobacterium tuberculosis. Antimicrob. Agents Chemother. 38, 805-811

36. Telenti, A., Imboden, P., Marchesi, F., Lowrie, D., Cole, S., Colston, M.J., Matter, L., Schopfer, K., and Bodmer, T. (1993). Detection of rifampicinresistance mutations in Mycobacterium tuberculosis. Lancet 341, 647-650

37. Kaufhold, A., Podbielski, A., Johnson, D.R., Kaplan, E.L., and Lütticken, R. (1992). M protein gene typing of Streptococcus pyogenes by nonradioactively labeled oligonucleotide probes. J. Clin. Microbiol. 30, 2391-2397

38. Colles, F.M., and Maiden, M.C.J. (2012). Campylobacter sequence typing databases: applications and future prospects. Microbiol. Read. Engl. 158, 2695-2709.

39. http://bioinformatics.net.au/software.velvetoptimiser.shtml

40. Altschul, S.F., Gish, W., Miller, W., Myers, E.W. & Lipman, D.J. (1990) "Basic local alignment search tool." J. Mol. Biol. 215:403-410. PubMed

41. http://jcm.asm.org/content/52/7/2479

42. http://jcm.asm.org/content/early/2014/04/17/JCM.00262-14.short

43. Hannes Pouseele, Bruno Pot, Setting up a genome-wide sequence typing scheme: how to overcome the pitfalls, in preparation.

**Claims**

1. A method for determining the presence or absence of one or more predefined nucleic acid sequences (referred to as alleles) in a set of nucleic acid sequences (referred to as read sequences), said method comprising:

   a) defining a k-mer, which is a nucleic acid sequence of a length "k" (for any natural number k), and a k-mer space which consists of all the permutations of nucleic acids ($4^k$) with the selected length "k", and wherein those nucleic acid sequences which are each other's reverse complement (that is, equal apart from their difference in direction, said direction being either forward (5'→3') or reverse (3'→5') direction), are considered as equivalent within the k-mer;
   b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;
   c) providing a set of read sequences;
   d) for each one or more predefined alleles and corresponding allele-associated k-mer sets, determining the number of occurrences of each k-mer (of the allele-associated k-mer set) in said set of read sequences, wherein those k-mers which are equivalent in the forward (5'→3') and reverse (3'→5') direction are defined as equivalent k-mers within the set of k-mers, but still can be discernable from each other if desired; wherein an occurrence of a k-mer is revokable based on suitable quality scores for individual nucleic acids in the read sequence;
   e) filtering the determined number of occurrences of each k-mer (of the allele-associated k-mer set) from step d); by resetting this number of occurrences of each k-mer to 0 if: i) the total number of occurrences (being either in the forward (5'→3') or reverse (3'→5') direction) is below a predefined threshold, ii) the total number of occurrences in the forward (5'→3') direction is below a predefined threshold, or iii) the total number of occurrences in the reverse (3'→5') direction is below a predefined threshold;
   f) determining the presence or absence of each predefined allele in the read sequences based on the filtered number of occurrences of each k-mer (of the allele-associated k-mer set) obtained from step e).

2. A method for determining the presence or absence of one or more sets (referred to as loci) containing one or more predefined nucleic acid sequences (referred to as alleles) in a set of nucleic acid sequences (referred to as read sequences), said method comprising:

   a) defining a k-mer, which is a nucleic acid sequence of a length "k" (for any natural number k), and a k-mer space which consists of all the permutations of nucleic acids ($4^k$) with the selected length "k", and wherein those nucleic acid sequences which are each other's reverse complement (that is, equal apart from their difference in direction, said direction being either forward (5'→3') or reverse (3'→5') direction), are considered as equivalent within the k-mer;
   b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;
   c) for each locus, determining which one or more allele-associated k-mer sets are present in a locus, and optionally determining the number of occurrences of each k-mer as defined in step a);
   d) providing a set of read sequences;
   e) for each locus and associated allele-associated k-mer sets, determining the presence of said one or more allele-associated k-mer sets and the number of occurrences of each k-mer (of each of the allele-associated k-mer sets) in said set of read sequences, wherein those k-mers which are equivalent in the forward (5'→3') and reverse (3'→5') direction are defined as equivalent k-mers within the set of k-mers, but still can be discernable from each other if desired; wherein an occurrence of a k-mer is revokable based on suitable quality scores for individual nucleic acids in the read sequence;
   f) filtering the determined number of occurrences of each k-mer (of each of the allele-associated k-mer sets) from step e); by resetting this number of occurrences of each k-mer to 0 if: i) the total number of occurrences (being either in the forward (5'→3') or reverse (3'→5') direction) is below a predefined threshold, ii) the total number of occurrences in the forward (5'→3') direction is below a predefined threshold, or iii) the total number of occurrences in the reverse (3'→5') direction is below a predefined threshold;
   g) determining the presence or absence of a loci containing one or more predefined alleles in the read sequences based on the filtered number of occurrences of each k-mer (of each of the allele-associated k-mer sets) obtained from step f).

3. A method for determining the presence or absence of one or more predefined amino acid sequences (referred to as alleles) in a set of amino acid sequences (referred to as read sequences), said method comprising:

a) defining a k-mer, which is an amino acid sequence of a length "k" (for any natural number k), and a k-mer space which consists of all the permutations of amino acids ($20^k$-$22^k$) with the selected length "k";

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) providing a set of read sequences;

d) for each one or more predefined alleles and corresponding allele-associated k-mer sets, determining the number of occurrences of each k-mer (of the allele-associated k-mer set) in said set of read sequences, wherein an occurrence of a k-mer is revokable based on suitable quality scores for individual amino acids in the read sequence;

g) filtering the determined number of occurrences of each k-mer (of the allele-associated k-mer set) from step d); by resetting this number of occurrences of each k-mer to 0 if the total number of occurrences is below a predefined threshold;

h) determining the presence or absence of each predefined allele in the read sequences based on the filtered number of occurrences of each k-mer(of the allele-associated k-mer set) obtained from step e).

4. A method for determining the presence or absence of one or more sets (referred to as loci) containing one or more predefined amino acid sequences (referred to as alleles) in a set of amino acid sequences (referred to as read sequences), said method comprising:

a) defining a k-mer, which is an amino acid sequence of a length "k" (for any natural number k), and a k-mer space which consists of all the permutations of amino acids ($20^k$-$22^k$) with the selected length "k";

b) for each one or more predefined alleles, determining which k-mer as defined in step a) is present in said each one or more predefined alleles, and optionally determining the number of occurrences of each k-mer as defined in step a) in said each one or more predefined alleles, thereby obtaining a corresponding allele-associated k-mer set;

c) for each locus, determining which one or more allele-associated k-mer sets are present in a locus, and optionally determining the number of occurrences of each k-mer as defined in step a);

d) providing a set of read sequences;

e) for each locus and associated allele-associated k-mer sets, determining the presence of said one or more allele-associated k-mer sets and the number of occurrences of each k-mer (of each of the allele-associated k-mer sets) in said set of read sequences, wherein an occurrence of a k-mer is revokable based on suitable quality scores for individual amino acids in the read sequence;

f) filtering the determined number of occurrences of each k-mer (of each of the allele-associated k-mer sets) from step e); by resetting this number of occurrences of each k-mer to 0 if the total number of occurrences (being either in the forward (5'→3') or reverse (3'→5') direction) is below a predefined threshold;

g) determining the presence or absence of a loci containing one or more predefined alleles in the read sequences based on the filtered number of occurrences of each k-mer(of each of the allele-associated k-mer sets) obtained from step f).

5. The method according to any one of claims 1 to 4, wherein in the case of absence of a predefined allele or of a loci containing one or more predefined aleles, the method further comprises determining the percentage of sequence identity of one or more of the k-mer when compared to the read sequences.

6. The method according to any one of claims 1 to 5, wherein the set of read sequences are unassembled, assembled, or partially assembled sequences.

7. The method according to claim 6, wherein the unassembled sequences are obtained from a sequencing platform selected from Sanger sequencing, pyrosequencing, sequencing by synthesis, and any other type that produces sequences of nucleic acids or amino acids.

8. The method according to claim 6, wherein the unassembled, assembled, or partially assembled sequences are obtained from any biological material, or in silico data.

9. The method according to claim 8, wherein the biological material is selected from one or more organisms and any part thereof.

10. The method according to claim 9, wherein the one more organisms is selected from prokariots including bacteria and archaea, from virus, fungi, microscopic arthropods, microscopic crustacean, any pathogenic, chimeric, or artificially-created microorganism, and any mixture thereof.

11. The method according to any one of claims 1 to 2, wherein the k is selected from 11 to 71 nucleic acids.

12. The method according to any one of claims 3 to 4, wherein the k is selected from 5 to 23 amino acids.

13. The method according to any one of claims 1 to 12, wherein the method is used for typing or subtyping; multi-locus sequence typing (MLST); extended multi-locus sequence typing (eMLST); ribosomal multi-locus sequence typing (rMLST); core genome sequence typing; whole genome multi-locus sequence typing (wgMLST, MLST+); spoligotyping for Mycobacterium Tuberculosis; large sequence polymorphism (LSP) detection for Mycobacterium Tuberculosis; Taqman®-based SNP analysis; single-locus sequence typing (or allelic typing); antibiotic resistance typing; antigen serotyping; SPA-typing (Serine Protease Autotransporters of Enterobacteriaceae); HIV, HBV, or HCV drug resistance prediction; DRU (direct-repeat unit) typing; mycobacterial interspersed repetitive units (MIRU) typing, variable number of tandem repeats (VNTR) typing, clustered regularly interspaced short palindromic repeats (CRISPR) typing.

14. A system for determining the presence or absence of one or more loci containing one or more predefined nucleic acid or amino acid sequences or variants thereof, or for determining the presence or absence of one or more predefined nucleic acid or amino acid sequences or variants thereof, in a set of read sequences, the system comprising at least one processor and an associated storage medium containing a program executable by means of said at least one processor, said system comprising software code portions executing the steps as defined in any one of claims 1 to 4, in any logical order.

15. The system of claim 14, comprising one or more of the following: a personal computer, a portable computer, a laptop computer, a netbook computer, a tablet computer, a smartphone, a digital still camera, a video camera, a mobile communication device, a personal digital assistant, a scanner, or a multi-function device.

16. A non-transient storage medium on which a computer program product is stored comprising software code portions in a format executable on a computer device and configured for performing the steps as defined in any one of claims 1 to 4, in any logical order, when executed on said computer device.

17. A computer program product executable on a computer device and comprising software code for executing the method according to any one of claims 1-13, when run on said computer device.

18. The non-transient storage medium according to claim 16 or the computer program product of claim 17, wherein the computer device is selected from a personal computer, a portable computer, a laptop computer, a netbook computer, a tablet computer, a smartphone, a digital still camera, a video camera, a mobile communication device, a personal digital assistant, a scanner, and a multi-function device.

Figure 1

Figure 2

Figure 3

Europäisches
Patentamt

European
Patent Office

Office européen
des brevets

# EUROPEAN SEARCH REPORT

Application Number

EP 15 15 3406

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/087412 A2 (APPLERA CORP [US]) 23 October 2003 (2003-10-23) * the whole document * | 1-18 | INV. G06F19/22 C12Q1/68 |
| X | US 2003/211504 A1 (FECHTEL KIM [US] ET AL) 13 November 2003 (2003-11-13) * the whole document * | 1-18 | |
| X | US 2013/345066 A1 (BRINZA DUMITRU [US] ET AL) 26 December 2013 (2013-12-26) * the whole document * | 1-18 | |
| A,D | JOLLEY KEITH A ET AL: "BIGSdb: Scalable analysis of bacterial genome variation at the population level", BMC BIOINFORMATICS, BIOMED CENTRAL, LONDON, GB, vol. 11, no. 1, 10 December 2010 (2010-12-10), page 595, XP021085869, ISSN: 1471-2105, DOI: 10.1186/1471-2105-11-595 * the whole document * | 1-18 | |
| A | WO 2004/099443 A2 (FEBIT AG [DE]; DAHMS MICHAEL [DE]; SCHLAUERSBACH ANDREA [DE]; BAUM MIC) 18 November 2004 (2004-11-18) * the whole document * | 1-18 | |

TECHNICAL FIELDS
SEARCHED (IPC)

G06F
C12Q

The present search report has been drawn up for all claims

| Place of search | Date of completion of the search | Examiner |
|---|---|---|
| Munich | 9 November 2015 | Mueller, Frank |

EPO FORM 1503 03.82 (P04C01)

## ANNEX TO THE EUROPEAN SEARCH REPORT
## ON EUROPEAN PATENT APPLICATION NO.

EP 15 15 3406

This annex lists the patent family members relating to the patent documents cited in the above-mentioned European search report.
The members are as contained in the European Patent Office EDP file on
The European Patent Office is in no way liable for these particulars which are merely given for the purpose of information.

09-11-2015

| Patent document cited in search report | | Publication date | Patent family member(s) | | Publication date |
|---|---|---|---|---|---|
| WO 03087412 | A2 | 23-10-2003 | AU<br>CA<br>EP<br>EP<br>JP<br>US<br>WO | 2003226109 A1<br>2481905 A1<br>1520246 A2<br>2175391 A2<br>2005522223 A<br>2003194724 A1<br>03087412 A2 | 27-10-2003<br>23-10-2003<br>06-04-2005<br>14-04-2010<br>28-07-2005<br>16-10-2003<br>23-10-2003 |
| US 2003211504 | A1 | 13-11-2003 | NONE | | |
| US 2013345066 | A1 | 26-12-2013 | NONE | | |
| WO 2004099443 | A2 | 18-11-2004 | EP<br>US<br>WO | 1620823 A2<br>2006241870 A1<br>2004099443 A2 | 01-02-2006<br>26-10-2006<br>18-11-2004 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **COHEN SH ; TANG YJ ; SILVA J JR.** Molecular typing methods for the epidemiological identification of Clostridium difficile strains. *Expert Rev Mol Diagn.,* 2001, vol. 1 (1), 61-70
- **SPRATT BG.** The 2011 Garrod Lecture: From penicillin-binding proteins to molecular epidemiology. *J Antimicrob Chemother,* 2012, vol. 67, 1578-1588
- **KNETSCH CW ; LAWLEY TD ; HENSGENS MP ; CORVER J ; WILCOX MW ; KUIJPER EJ.** Current application and future perspectives of molecular typing methods to study Clostridium difficile infections. *Euro Surveill.,* 2013, vol. 18 (4), 20381
- **VOTH DE ; BALLARD JD.** Clostridium difficile toxins: mechanism of action and role in disease. *Clin Microbiol Rev.,* 2005, vol. 18 (2), 247-63
- **ISON, C. A. et al.** International comparison of molecular typing methods for Neisseria gonorrhoeae. *15th Int. Soc. Sex. Transm. Dis. Res. Congr.,* 2003
- **PÉREZ-LOSADA, M. ; K. A. CRANDALL ; J. ZENILMAN ; R. P. VISCIDI.** Temporal trends in gonococcal population genetics in a high prevalence urban community. *Infect. Genet. Evol.,* 2007, vol. 7, 271-278
- **PÉREZ-LOSADA, M. et al.** Distinguishing importation from diversification of quinolone-resistant Neisseria gonorrhoeae by molecular evolutionary analysis. *BMC. Evol. Biol.,* 2007, vol. 7, 84
- **PÉREZ-LOSADA, M. ; R. P. VISCIDI ; J. C. DEMMA ; J. ZENILMAN ; K. A. CRANDALL.** Population genetics of Neisseria gonorrhoeae in a highprevalence community using a hypervariable outer membrane porB and 13 slowly evolving housekeeping genes. *Mol. Biol. Evol.,* 2005, vol. 22, 1887-1902
- **TAZI, L. et al.** Population dynamics of Neisseria gonorrhoeae in Shanghai, China: a comparative study. *BMC Infect. Dis.,* 2010, vol. 10, 13
- **MAGNUS UNEMO ; JO-ANNE R. DILLON.** Review and International Recommendation of Methods for Typing Neisseria gonorrhoeae Isolates and Their Implications for Improved Knowledge of Gonococcal Epidemiology, Treatment, and Biology. *Clinical Microbiology Reviews,* July 2011, 447-458
- **INOUYE M ; CONWAY T ; ZOBEL J ; HOLT KE.** Short read sequence typing (SRST): multi-locus sequence types from short reads. *BMC Genomics,* 2012, vol. 13, 338

- **COLL F ; MALLARD K ; PRESTON M ; BENTLEY S ; PARKHILL J ; MCNERNEY R ; MARTIN N ; CLARK T.** SpolPred: rapid and accurate prediction of Mycobacterium tuberculosis spoligotypes from short genomic sequences. *Bioinformatics,* 15 November 2012, vol. 28 (22), 2991-2993
- **JOLLEY K.A. ; MAIDEN M.C.** BIGSdb: Scalable analysis of bacterial genome variation at the population level. *BMC Bioinformatics,* 10 December 2010, vol. 11, 595
- **HUNTER PR ; GASTON MA.** Numerical index of the discriminatory ability of typing systems: an application of Simpson's index of diversity. *J Clin Microbiol.,* 1988, vol. 26 (11), 2465-6
- **VAN BELKUM A ; TASSIOS PT ; DIJKSHOORN L ; HAEGGMAN S ; COOKSON B ; FRY NK et al.** Guidelines for the validation and application of typing methods for use in bacterial epidemiology. *Clin Microbiol Infect.,* 2007, vol. 13 (3), 1-46
- **NADON CA ; TREES E ; NG LK ; MØLLER NIELSEN E ; REIMER A ; MAXWELL N ; KUBOTA KA ; GERNER-SMIDT P.** the MLVA Harmonization Working Group. Development and application of MLVA methods as a tool for interlaboratory surveillance. *Euro Surveill.,* 2013, vol. 18 (35), 20565
- **MAIDEN, M.C.J. ; BYGRAVES, J.A. ; FEIL, E. ; MORELLI, G. ; RUSSELL, J.E. ; URWIN, R. ; ZHANG, Q. ; ZHOU, J. ; ZURTH, K. ; CAUGANT, D.A. et al.** Multilocus sequence typing: A portable approach to the identification of clones within populations of pathogenic microorganisms. *Proc. Natl. Acad. Sci. U. S. A.,* 1998, vol. 95, 3140-3145
- **DIDELOT, X. ; URWIN, R. ; MAIDEN, M.C.J. ; FALUSH, D.** Genealogical typing of Neisseria meningitidis. *Microbiology,* 2009, vol. 155, 3176-3186
- **JOLLEY, K.A. ; BLISS, C.M. ; BENNETT, J.S. ; BRATCHER, H.B. ; BREHONY, C. ; COLLES, F.M. ; WIMALARATHNA, H. ; HARRISON, O.B. ; SHEPPARD, S.K. ; CODY, A.J. et al.** Ribosomal multilocus sequence typing: universal characterization of bacteria from domain to strain. *Microbiol. Read. Engl.,* 2012, vol. 158, 1005-1015
- **CODY, A.J. ; MCCARTHY, N.D. ; RENSBURG, M.J. VAN ; ISINKAYE, T. ; BENTLEY, S. ; PARKHILL, J. ; DINGLE, K.E. ; BOWLER, I.C.J.W. ; JOLLEY, K.A. ; MAIDEN, M.C.J.** Real-time genomic epidemiology of human Campylobacter isolates using whole genome multilocus sequence typing. *J. Clin. Microbiol.,* 2013

- **JOLLEY, K.A. ; HILL, D.M.C. ; BRATCHER, H.B. ; HARRISON, O.B. ; FEAVERS, I.M. ; PARKHILL, J. ; MAIDEN, M.C.J.** Resolution of a Meningococcal Disease Outbreak from Whole-Genome Sequence Data with Rapid Web-Based Analysis Methods. *J. Clin. Microbiol.,* 2012, vol. 50, 3046-3053
- **GROENEN, P.M.A. ; BUNSCHOTEN, A.E. ; SOOL-INGEN, D. VAN ; ERRTBDEN, J.D.A. VAN.** Nature of DNA polymorphism in the direct repeat cluster of Mycobacterium tuberculosis; application for strain differentiation by a novel typing method. *Mol. Microbiol.,* 1993, vol. 10, 1057-1065
- **KAMERBEEK, J. ; SCHOULS, L. ; KOLK, A. ; AGTERVELD, M. VAN ; SOOLINGEN, D. VAN ; KU-IJPER, S. ; BUNSCHOTEN, A. ; MOLHUIZEN, H. ; SHAW, R ; GOYAL, M. et al.** Simultaneous detection and strain differentiation of Mycobacterium tuberculosis for diagnosis and epidemiology. *J. Clin. Microbiol.,* 1997, vol. 35, 907-914
- **GAGNEUX, S. ; DERIEMER, K. ; VAN, T. ; KA-TO-MAEDA, M. ; DE JONG, B.C. ; NARAYANAN, S. ; NICOL, M. ; NIEMANN, S. ; KREMER, K. ; GU-TIERREZ, M.C. et al.** Variable host-pathogen compatibility in Mycobacterium tuberculosis. *Proc. Natl. Acad. Sci. U. S. A.,* 2006, vol. 103, 2869-2873
- **KATO-MAEDA, M. ; RHEE, J.T. ; GINGERAS, T.R. ; SALAMON, H. ; DRENKOW, J. ; SMITTIPAT, N. ; SMALL, P.M.** Comparing Genomes within the Species Mycobacterium tuberculosis. *Genome Res.,* 2001, vol. 11, 547-554
- **TSOLAKI, A.G. ; HIRSH, A.E. ; DERIEMER, K. ; ENCISO, J.A. ; WONG, M.Z. ; HANNAN, M. ; SAL-MONIERE, Y.-O.L.G. DE LA ; AMAN, K. ; KA-TO-MAEDA, M. ; SMALL, P.M.** Functional and evolutionary genomics of Mycobacterium tuberculosis: Insights from genomic deletions in 100 strains. *Proc. Natl. Acad. Sci. U. S. A.,* 2004, vol. 101, 4865-4870
- **HIRSH, A.E. ; TSOLAKI, A.G. ; DERIEMER, K. ; FELDMAN, M.W. ; SMALL, P.M.** Stable association between strains of Mycobacterium tuberculosis and their human host populations. *Proc. Natl. Acad. Sci. U. S. A.,* 2004, vol. 101, 4871-4876
- **FLEISCHMANN, R.D. ; ALLAND, D. ; EISEN, J.A. ; CARPENTER, L. ; WHITE, O. ; PETERSON, J. ; DE-BOY, R. ; DODSON, R. ; GWINN, M. ; HAFT, D. et al.** Whole-Genome Comparison of Mycobacterium tuberculosis Clinical and Laboratory Strains. *J. Bacteriol.,* 2002, vol. 184, 5479-5490
- **MILLER, L.P. ; CRAWFORD, J.T. ; SHINNICK, T.M.** The rpoB gene of Mycobacterium tuberculosis. *Antimicrob. Agents Chemother,* 1994, vol. 38, 805-811
- **TELENTI, A. ; IMBODEN, P. ; MARCHESI, F. ; LOWRIE, D. ; COLE, S. ; COLSTON, M.J. ; MAT-TER, L. ; SCHOPFER, K. ; BODMER, T.** Detection of rifampicinresistance mutations in Mycobacterium tuberculosis. *Lancet,* 1993, vol. 341, 647-650
- **KAUFHOLD, A. ; PODBIELSKI, A. ; JOHNSON, D.R. ; KAPLAN, E.L. ; LÜTTICKEN, R.** M protein gene typing of Streptococcus pyogenes by nonradioactively labeled oligonucleotide probes. *J. Clin. Microbiol.,* 1992, vol. 30, 2391-2397
- **COLLES, F.M. ; MAIDEN, M.C.J.** Campylobacter sequence typing databases: applications and future prospects. *Microbiol. Read. Engl.,* 2012, vol. 158, 2695-2709
- **ALTSCHUL, S.F. ; GISH, W. ; MILLER, W. ; MYE-RS, E.W. ; LIPMAN, D.J.** Basic local alignment search tool. *J. Mol. Biol.,* 1990, vol. 215, 403-410